Europäisches Patentamt

European Patent Office

Office européen des brevets

(19)

(11) **EP 1 043 019 A1**

(12) ## DEMANDE DE BREVET EUROPEEN

(43) Date de publication:
**11.10.2000 Bulletin 2000/41**

(51) Int Cl.⁷: **A61K 7/50**, A47L 13/17

(21) Numéro de dépôt: **00400888.4**

(22) Date de dépôt: **31.03.2000**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **09.04.1999 FR 9904442**

(71) Demandeur: **Laboratoires Prodene Klint
77290 Mitry-Mory (FR)**

(72) Inventeur: **Vignot, Eric,
Laboratoires Prodene Klint
77290 Mitry Mory (FR)**

(74) Mandataire: **Boutin, Antoine
Cabinet Tony-Durand,
78, avenue Raymond Poincaré
75116 Paris (FR)**

(54) **Article de nettoyage utilisé hors proximité d'un point d'eau**

(57)    L'article de nettoyage utilisé hors proximité d'un point d'eau, comprend un substrat abrasif et un liquide d'imprégnation. Le substrat est un non-tissé abrasif dans la masse et le liquide d'imprégnation est une solution aqueuse de d-limonène, de tensioactifs et de solvants hydrophiles avec éventuellement des additifs usuels.

EP 1 043 019 A1

**Description**

L'invention concerne un article de nettoyage, en particulier des mains, utilisé hors proximité d'un point d'eau, comprenant un substrat abrasif et un liquide d'imprégnation.

US-A-4.833.003 décrit un empilement de serviettes abrasives humides, les serviettes ayant au moins une surface abrasive et contenant une solution de nettoyage quelconque. La surface abrasive est obtenue par projection d'un polymère fondu sur un non-tissé de polyoléfines.

US-A-5.683.971 décrit des articles abrasifs de nettoyage des mains, comprenant un substrat selon le document précédent, et une émulsion de nettoyage des mains imprégnée dans le substrat et comprenant 2 à 40 % d'un solvant organique émulsifiable, 2 à 20 % d'un tensio-actif pouvant former une émulsion eau/huile, et 60 à 95 % d'eau.

Les non-tissés abrasifs utilisés dans ces deux documents ont un aspect et une qualité de surface très hétérogène qui les rendent agressifs vis-à-vis des mains et qui posent des problèmes au niveau de leur manipulation, et notamment au niveau des machines de bobinage et de débobinage par la découpe des serviettes car les amas de polyoléfine peuvent provoquer leur grippage.

L'émulsion de nettoyage utilisée dans le deuxième document de la technique antérieure contient des solvants organiques emulsifiables, comme de l'essence minérale, qui sont irritants. En outre, l'émulsion est instable : par gravité, il y a séparation avec concentration des solvants agressifs et on est donc contraint de mélanger continuellement l'émulsion pendant l'imprégnation des non-tissés.

L'utilisation d'une émulsion impose également l'utilisation d'un conteneur spécifique, à savoir une boîte "cheminée" contenant un rouleau de serviettes reliées en continu avec une cheminée centrale pour l'imprégnation de l'émulsion.

L'invention permet de surmonter ces inconvénients en fournissant un article de nettoyage, notamment mais pas exclusivement des mains, destiné à être utilisé hors proximité d'un point d'eau, qui ne soit pas agressivement abrasif, qui ne contienne pas de solvants irritants, qui ne nécessite pas un mélange continu du liquide d'imprégnation et qui ne soit pas limité à une seule présentation de fabrication et d'utilisation.

L'article de nettoyage est ainsi constitué d'un substrat qui est un non-tissé abrasif dans la masse et le liquide d'imprégnation est une solution aqueuse de d-limonène, de tensio-actifs et de solvants hydrophiles avec éventuellement des additifs usuels.

Le non-tissé abrasif dans la masse est obtenu par utilisation d'un mélange de libres abrasives choisies parmi des fibres naturelles, artificielles ou synthétiques ou mixtes par exemple des fibres de polyoléfine, par exemple polyéthylène ou polypropylène ; Ces fibres sont transformées en un substrat abrasif sur les deux faces par un procédé classique de fabrication de non-tissé. Ces fibres sont éventuellement teintées dans la masse si on souhaite que la serviette finale ait une couleur particulière. Un produit de ce type est disponible chez AHLSTROM sous la dénomination SPUNLACE et est constitué d'un mélange de 85 % en poids de fibre de cellulose (40 %), viscose (10 %) et polyester (35 %), et de 15 % de liants (acrylique ou autres).

La solution d'imprégnation contient essentiellement en solution aqueuse du d-limonène, des tensio-actifs et des solvants hydrophiles. Elle peut également contenir des additifs usuels, comme des parfums et des conservateurs.

Le ratio d'imprégnation est compris entre 1,5 et 4 ; on entend par ratio le rapport

$$\sim = \frac{\text{masse de la solution}}{\text{masse du non-tissé}}$$

Les tensio-actifs peuvent être des tensio-actifs non-ioniques, amphotères, anioniques ou cationiques. On peut citer à titre d'exemples les suivants :

- anioniques, LES Na (TEXAPON-HENKEL) : alkylsulfosuccinate et autres,
- non ioniques, (EUMULGIN-HENKEL) : alcools gras et corps gras éthoxylés et autres,
- amphotères, (TEGOBETAINE- GOLDSCHMIDT) : bétaïnes, propioniques et autres,
- cationiques (CATIGENE-STEPHAN): ammoniums quaternaires, dérivés et autres.

Les solvants organiques hydrophiles qui aident à la solubilisation du d-limonène sont choisis parmi les alcools (méthanol, éthanol, ou alcools supérieurs), les glycols (propylèneglycol et autres), les esters organiques et en particulier les esters dibutylique des acides glutarique, adipide, butyrique, etc...

Le tableau suivant donne la gamme de compositions permettant d'obtenir une solution aqueuse limpide, stable, et sans aucun déphasage des constituants dans le temps, ce qui montre qu'il s'agit d'une vraie solution.

| Ingrédients | Gamme large | Gamme étroite | Préférée |
|---|---|---|---|
| d-Limonène | 0,05 à 0,9 % | 0,5 à 0,9 % | 0,9 % |
| Tensio-actifs | 2 à 40 % | 5 à 20 % | 10 % |
| Solvants organiques hydrophiles | 8 à30 % | 8 à 15 % | 9 % |
| Conservateur | 0,02 à 1,5% | 0,1 à 1 % | 0,5 % |
| Parfum | 0,1 à 1 | 0,1 à 0,5% | 0,2 % |
| Eau | 26 à 90 | 64 à 87 % | 79,4 % |
| % en poids de la solution. | | | |

Exemple de préparation

On prépare une solution selon la composition préférée du tableau ci-dessus.

Par ailleurs, on prépare une pile de serviettes à partir d'une bobine mère de non-tissé Spunlace, de dimensions 200 x 350 mm dans une boîte de conditionnement de dimensions correspondantes. La pile de pièces de non-tissé a un poids de 300 g.

Sur cette pile, on verse en fonction du ratio d'imprégnation défini ci-dessus la solution préalablement préparée, sans qu'il soit nécessaire de l'agiter et on les laisse se diffuser dans toute la pile de serviettes. Au bout de quelques minutes, les serviettes sont uniformément imprégnées de la solution et cette imprégnation reste stable dans le temps.

Le procédé d'imprégnation d'un rouleau continu de serviettes dans une boîte cheminée peut bien entendu être utilisée avec la solution selon l'invention, si on souhaite utiliser une présentation et une distribution de ce type.

La possibilité d'imprégner les serviettes à plat permet une présentation à plat qui autorise un conditionnement en petites quantités ou même individuel.

Il s'est avéré que la serviette ainsi obtenue, qui ne contient pas de solvants du type essence minérale susceptibles d'attaquer les matières plastiques, peut être utilisée non seulement pour le nettoyage des mains tâchées d'huiles, goudrons, peintures fraîches, encres, tâches d'encre par stylos-feutre, traces d'herbe, ..., mais aussi pour le nettoyage de diverses surfaces sensibles du type carcasses de machines de bureau et notamment d'ordinateurs, ou de divers revêtements de sols et de murs.

## Revendications

1. Article de nettoyage utilisé hors proximité d'un point d'eau, comprenant un substrat abrasif et un liquide d'imprégnation, caractérisé en ce que le substrat est un non-tissé abrasif dans la masse et le liquide d'imprégnation est une solution aqueuse qui contient de 0,05 à 0,9 % en poids de d-limonène, de 2 à 40 % de tensio-actifs, de 8 à 30 % de solvants hydrophiles, de 0,02 à 1,5 % de conservateurs, de 0,1 à 1 % de parfum et de 26 à 90 % d'eau (en % en poids de la solution).

2. Article de nettoyage selon la revendication 1, caractérisé en ce que le non-tissé abrasif dans la masse est constitué d'un mélange de fibres abrasives éventuellement teintées dans la masse.

3. Article de nettoyage des mains selon la revendication 2, caractérisé en ce que les fibres abrasives sont choisies parmi les fibres naturelles, artificielles ou synthétiques ou mixtes.

4. Article de nettoyage selon l'une quelconque des revendications 1 à 3, caractérisé en ce que la solution aqueuse d'inifriguation contient de 0,5 à 0,9 % de d-limonène, de 5 à 20 % de tensio-actifs, de 8 à 15 % de solvants hydrophiles, de 0,1 à 1 % de conservateurs, de 0,1 à 0,5 % de parfum et de 64 à 87 % d'eau.

5. Article de nettoyage selon l'une quelconque des revendications 1 à 4, caractérisé en ce que la solution aqueuse d'imprégnation contient 0,9 % de d-limonène, 10 % de tensio-actifs, 9 % de solvants hydrophiles, 0,5 % de conservateurs, 0,2 % de parfum et 79,4 % d'eau.

6. Article de nettoyage selon l'une quelconque des revendications 4 et 5, caractérisé en ce que les solvants hydrophiles sont choisis parmi les alcools, les glycols, les esters organiques et le glycérol.

7. Article de nettoyage selon l'une quelconque des revendications 4 à 6, caractérisé en ce que les tensio-actifs sont choisis parmi les tensio-actifs non ioniques, amphotères, cationiques ou anioniques.

**8.** Article de nettoyage selon l'une quelconque des revendications 1 à 7, sous forme d'une serviette.

**9.** Empilement d'articles de nettoyage sous forme de serviettes selon la revendication 8 dans un conteneur distributeur.

**10.** Procédé de réalisation d'un empilement d'articles de nettoyage selon la revendication 9, caractérisé en ce qu'on empile à plat des morceaux de non-tissé abrasif dans la masse aux dimensions de la serviette finale et on les imprègne en y versant la solution aqueuse d'imprégnation selon un ratio d'imprégnation compris entre 1,5 et 4.

**EP 1 043 019 A1**

Office européen
des brevets

**RAPPORT DE RECHERCHE EUROPEENNE**

Numéro de la demande

EP 00 40 0888

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | US 5 891 835 A (VLASBLOM JACK T) 6 avril 1999 (1999-04-06) * revendications * | 1-3,8 | A61K7/50 A47L13/17 |
| D,A | US 5 683 971 A (MIKE ANDREW V ET AL) 4 novembre 1997 (1997-11-04) * revendications * | 1-3,8-10 | |
| A | US 5 320 772 A (TRICCA R EUGENE) 14 juin 1994 (1994-06-14) * revendications 5,6; figures * | 1,8-10 | |
| A | GB 618 075 A (PIETERKOWSKI MAX) 16 février 1949 (1949-02-16) * le document en entier * | 1 | |

**DOMAINES TECHNIQUES RECHERCHES (Int.Cl.7)**

C11D
A61K

Le présent rapport a été établi pour toutes les revendications

| Lieu de la recherche | Date d'achèvement de la recherche | Examinateur |
|---|---|---|
| LA HAYE | 21 juillet 2000 | Grittern, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

5

**ANNEXE AU RAPPORT DE RECHERCHE EUROPEENNE
RELATIF A LA DEMANDE DE BREVET EUROPEEN NO.**

EP 00 40 0888

La présente annexe indique les membres de la famille de brevets relatifs aux documents brevets cités dans le rapport de recherche européenne visé ci–dessus.
Lesdits members sont contenus au fichier informatique de l'Office européen des brevets à la date du
Les renseignements fournis sont donnés à titre indicatif et n'engagent pas la responsabilité de l'Office européen des brevets.

21-07-2000

| Document brevet cité au rapport de recherche | | Date de publication | Membre(s) de la famille de brevet(s) | | Date de publication |
|---|---|---|---|---|---|
| US 5891835 | A | 06-04-1999 | AUCUN | | |
| US 5683971 | A | 04-11-1997 | AT | 184171 T | 15-09-1999 |
| | | | AU | 674919 B | 16-01-1997 |
| | | | AU | 5785594 A | 22-09-1994 |
| | | | CA | 2117243 A | 19-09-1994 |
| | | | CN | 1097300 A | 18-01-1995 |
| | | | DE | 69420441 D | 14-10-1999 |
| | | | DE | 69420441 T | 27-01-2000 |
| | | | EP | 0615720 A | 21-09-1994 |
| | | | ES | 2136162 T | 16-11-1999 |
| | | | GR | 3031609 T | 31-01-2000 |
| | | | IL | 109002 A | 08-02-1998 |
| | | | JP | 7053352 A | 28-02-1995 |
| US 5320772 | A | 14-06-1994 | AUCUN | | |
| GB 618075 | A | | AUCUN | | |

EPO FORM P0460

Pour tout renseignement concernant cette annexe : voir Journal Officiel de l'Office européen des brevets, No.12/82